# EUROPEAN PATENT APPLICATION

(11) **EP 1 916 020 A2**
(43) Date of publication of application: **30.04.2008**
(21) Application number: 08001023.4
(22) Date of filing: 14.08.1998
(51) Int. Cl.: A61P 37/06, A61K 39/395

(54) **Preventives and/or remedies for systemic lupus erythematosus containing anti-IL-6 receptor antibody as the active ingredient**

(30) Priority: 15.08.1997 JP 22040097
(62) Divisional of application: 98937834.4
(71) Applicant: CHUGAI SEIYAKU KABUSHIKI KAISHA, Tokyo, 115-8543 (JP)
(72) Inventor: The designation of the inventor has not yet been filed
(74) Representative: Wakerley, Helen Rachael

(57) **Abstract**

A preventive and/or therapeutic agent for systemic lupus erythematosus comprising an anti-interleukin-6 (IL-6) receptor antibody as an active ingredient.

## Description

### Technical Field

The present invention relates to a preventive and/or therapeutic agent for systemic lupus erythematosus comprising an anti-interleukin-6 receptor antibody as an active ingredient.

### Background Art

Interleukin-6 (IL-6) is a cytokeine which is also called B cell stimulating factor 2 (BSF2) or interferon β2 (IFN-β2). IL-6 was discovered as a differentiation factor involved in the activation of B lymphocytes (Hirano, T. et al., Nature (1986) 324, 73-76). Thereafter, it was found to be a multifunctional cytokeine that influences various functions of the cells (Akira, S. et al., Adv. in Immunology (1993) 54, 1-78).

IL-6 transmits its biological activity through two types of proteins on the cell. One is IL-6 receptor, a ligand-biding protein to which IL-6 binds. IL-6 receptor occurs not only as a membrane-bound IL-6 receptor, molecular weight of about 80 kD, that penetrates through and is expressed on the cell membrane but also as a soluble IL-6 receptor, molecular weight of about 40 to 50 kD, essentially consisting of the extracellular region.

The other is a membrane protein gp130, molecular weight of about 130 kD, that is involved in the non-ligand-biding signal transduction. IL-6 and IL-6 receptor form the IL-6/IL-6 receptor complex, which after binding to gp130 transmits its biological activity into the cell (Taga et al., J. Exp. Med. (1987) 166, 967).

Anti-IL-6 receptor antibody has been described in several reports (Novick D. et al., Hybridoma (1991) 10, 137-146, Huang, Y. W. et al., Hybridoma (1993) 12, 621-630, International Patent Publication WO 95-09873, French Patent Publication FR 2694767, United States Patent US 521628). A mouse antibody PM-1 (Hirata et al., J. Immunology (1989) 143, 2900-2906) that is one of the anti-interleukin-6 receptor antibodies and humanized PM-1 antibody (the International Patent Publication WO 92-19759) have been known, and the latter was obtained by transplanting the complementarity determining region (CDR) thereof to a human antibody.

Systemic lupus erythematosus is a systemic autoimmune disease that results from inflammatory reactions caused by the deposition of antinuclear antibody or anti-DNA antibody and their immune complexes in various organs and tissues. The deposition of antinuclear antibody or anti-DNA antibody and their immune complexes in the renal glomerulus triggers the onset of lupus nephritis. In systemic lupus erythematosus, few patients exhibit increased IL-6 levels in the blood, negating the involvement of IL-6 in the pathology (Peterson, E. et al., Lupus. (1996) 5, 571-576).

Kiberd, J., J. Am. Soc. Nephrol. (1993) 4, 58-61, describes the effect of anti-interleukin-6 receptor antibody on renal functions and tissue damage in MRL-lpr/lpr mice. In the experiment by Kiberd, L., however, no investigations were made on the timing of appearance of urinary protein and days of survival as an index of clinical symptoms of systemic lupus erythematosus. Thus, their results alone cannot conclusively assert that anti-interleukin-6 receptor antibody is effective for lupus nephritis.

Though the pathology of the MRL-lpr/lpr mice used is similar histologically to systemic lupus erythematosus, the onset mechanism of the pathology involves an aberrant Fas antigen. On the other hand, no aberrant Fas antigens have been observed in human systemic lupus erythematosus (Watanabe-Fukunaga, R. et al., Nature, (1992) 356, 314-317).

It is therefore difficult to believe that the results obtained in these mice could immediately be applied to systemic lupus erythematosus in humans. As models of lupus nephritis in systemic lupus erythematosus, NZB/WF1 mice are known to have a pathology closest to that of systemic lupus erythematosus in humans (Howie, J. B. et al., Adv. Immunol. (1968) 9, 215-266).

In addition, since there were no statistically significant differences observed between the anti-interleukin-6 receptor antibody administration group and the control IgG administration group, it cannot be concluded that anti-interleukin-6 receptor antibody has any therapeutic effects. Thus, from the above literature by Kiberd. J., one could not expect the possible effects of anti-interleukin-6 receptor antibody on systemic lupus erythematosus.

Finck, B. K. et al., J. Clin. Invest. (1994) 94, 585-591 described the suppressive effect on the excretion of urinary protein, the suppressive effects on ant-DNA antibody production in the blood and the prolongation of survival days in anti-IL-6 antibody-treated NZB/NZB F1 mice.

However, the administration of anti-IL-6 antibody resulted in an increased number of mice that developed nephritis from 7-month-old, and it is very likely that the increase continues even after 9-month-old. It is thus anticipated that anti-IL-6 antibody has no sufficient effect on systemic lupus erythematosus.

It has been reported to date that the administration of anti-IL-6 antibody in man and mice causes marked increases in blood levels of IL-6 (Wendling, D. et al., J. Rheumatol. (1993) 20, 259-262; Heremans, H. et al., Eur. J. Immunol. (1992) 22, 2395-2401). It has also been found that the simultaneous administration of IL-6 and anti-IL-6 antibody results in marked increases in the activity of IL-6 (Mihara, M. et al., Immunology (1991) 74, 55-59). These facts indicate the possibility that the use of anti-IL-6 antibody causes an enhancement of the endogenous activity of IL-6 and the development of side-effects. It is suggested therefore that undesirable effects may arise from the administration of anti-IL-6 antibody to patients with systemic lupus erythematosus.

International Patent Publication WO 96-12503 describes that anti-IL-6 receptor antibody is effective for nephritis, in particular mesangial proliferative nephritis. However, the International Patent Publication WO 96-12503 only describes that IL-6 produced in large quantities by genetic engineering/induced the growth of mesangial cells, and nephritis developed from the growth of mesangial cells as a direct cause. It makes no description or suggestion that IL-6 is not involved in the onset, and that anti-IL-6 receptor antibody has any therapeutic effects on autoimmune nephritis, for example lupus nephritis, in systemic lupus erythematosus that is caused by the deposition in the renal glomerulus of anti-nuclear antibody or anti-DNA antibody or their immune complexes.

Accordingly, it has not been known that anti-IL-6 receptor antibody has therapeutic effects on systemic lupus erythematosus.

Currently, systemic lupus erythematosus has been treated by steroid drugs or/and immunosuppressive drugs. However, they are symptomatic therapies, and they require a long-term administration and pose problems of side-effects.

### Disclosure of the Invention.

The present invention intends to provide a preventive and/or therapeutic agent for systemic lupus erythematosus, said agent being free of the above-mentioned drawbacks.

The present invention provides a preventive and/or therapeutic agent for systemic lupus erythematosus comprising an anti-IL-6 receptor antibody as an active ingredient.

The present invention also provides a preventive and/or therapeutic agent for autoimmune nephritis in systemic lupus erythematosus comprising an anti-IL-6 receptor antibody as an active ingredient.

The present invention also provides a preventive and/or therapeutic agent for lupus nephritis in systemic lupus erythematosus comprising an anti-IL-6 receptor antibody as an active ingredient.

The present invention provides a preventive and/or therapeutic agent for the above systemic lupus erythematosus comprising an anti-human IL-6 receptor antibody as an active ingredient.

The present invention provides a preventive and/or therapeutic agent for the above systemic lupus erythematosus comprising an anti-IL-6 receptor monoclonal antibody as an active ingredient.

The present invention provides a preventive and/or therapeutic agent for the above systemic lupus erythematosus comprising an recombinant anti-IL-6 receptor antibody as an active ingredient.

The present invention provides a preventive and/or therapeutic agent for the above systemic lupus erythematosus comprising PM-1 antibody as an active ingredient.

The present invention provides a preventive and/or therapeutic agent comprising an anti-IL-6 receptor antibody having the constant region of a human antibody as an active ingredient.

The present invention also provides a preventive and/or therapeutic agent for systemic lupus erythematosus comprising a chimeric antibody or a humanized antibody directed against IL-6 receptor as an active ingredient.

The present invention also provides a preventive and/or therapeutic agent for systemic lupus erythematosus comprising a humanized PM-1 antibody as an active ingredient.

The present invention also provides a preventive and/or therapeutic agent for reducing anti-DNA antibody or anti-nuclear antibody in systemic lupus erythematosus comprising an anti-IL-6 receptor antibody as an active ingredient.

The present invention also provides a preventive and/or therapeutic agent for reducing the excretion of urinary protein in systemic lupus erythematosus comprising an anti-IL-6 receptor antibody as an active ingredient.

### Brief Description of Drawings

Fig. 1 is a graph showing the suppressive effect on the excretion of urinary protein by MR16-1 in NZB/W F1 mice.

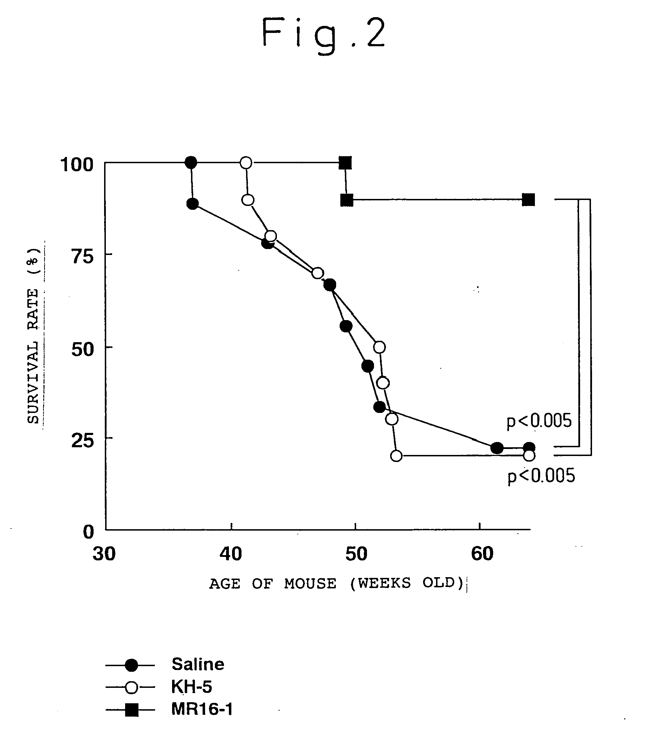

Fig. 2 is a graph showing the effect on survival days by MR16-1 in NZB/W F1 mice.
Fig. 3 is a graph showing the effect on the amount of anti-DNA antibody in the blood by MR16-1. Embodiments for Carrying Out the Invention

### 1. Anti-IL-6 receptor antibody

Anti-IL-6 receptor antibodies for use in the present invention may be of any origin, any kind, and any form, as long as it has a therapeutic effect for systemic lupus erythematosus, an effect of reducing anti-DNA antibody in systemic lupus erythematosus, or an effect of reducing the excretion of urinary protein in systemic lupus erythematosus.

Anti-IL-6 receptor antibodies for use in the present invention are antibodies that inhibit the binding of IL-6 to IL-6 receptor by binding to IL-6 receptor, thereby inhibiting the transmission of the biological activity of IL-6 into the cells. Accordingly, anti-IL-6 receptor antibodies for use in the present invention are preferably antibodies that neutralize the biological activity of IL-6.

Anti-IL-6 receptor antibodies for use in the present invention can be obtained as polyclonal or monoclonal antibodies using a known method. As the anti-IL-6 receptor antibodies for use in the present invention, monoclonal antibodies are preferred. Furthermore, as the anti-IL-6 receptor antibodies, monoclonal antibodies of, in particular, mammalian origin, are preferred.

Monoclonal antibodies of mammalian origin include those produced by a hybridoma and those produced by a host which has been transformed with an expression vector containing genetically engineered antibody genes.

Examples of such antibodies include MR16-1 antibody (Saito, et al., J. Immunology (1993) 147, 168-173), PM-1 antibody (Hirata, et al., J. Immunology (1989) 143, 2900-2906), or AUK12-20 antibody, AUK64-7 antibody or AUK146-15 antibody (International Patent Publication WO 92-19759), and the like. Among them, PM-1 antibody is most preferred.

Incidentally, the hybridoma cell line which produces PM-1 antibody has been internationally deposited under the provisions of the Budapest Treaty on July 10, 1990 with the National Institute of Bioscience and Human Technology, Agency of Industrial Science and Technology, of 1-3, Higashi 1-chome, Tsukuba-shi, Ibaraki, Japan, as FERM BP-2998.

The hybridoma cell line which produces MR16-1 antibody has been internationally deposited under the provisions of the Budapest Treaty as MR16-1 on March 13, 1997 with the National Institute of Bioscience and Human Technology, Agency of Industrial Science and Technology, of 1-3, Higashi 1-chome, Tsukuba-shi, Ibaraki, Japan, as FERM BP-5875.

### 2. Preparation of IL-6 receptor

IL-6 receptor which is an immunizing antigen may be prepared using a known method.

Specifically, IL-6 receptor can be obtained in the following manner. For example, human IL-6 receptor can be obtained using the IL-6 receptor gene sequence/amino acid sequence disclosed in European Patent Publication EP 325474, and the mouse IL-6 receptor can be obtained using that disclosed in Japanese Unexamined Patent Publication (Kokai) 3-155795. The IL-6 receptor to be used as an immunizing antigen for generating antibody is preferably human IL-6 receptor.

There are two types of IL-6 receptor proteins: IL-6 receptor expressed on the cell membrane, and IL-6 receptor detached from the cell membrane (soluble IL-6 Receptor) (Yasukawa et al., J. Biochem. (1990) 108, 673-676). Soluble IL-6 receptor antibody essentially consists of the extracellular region of the IL-6 receptor bound to the cell membrane, and thereby is different from the membrane-bound IL-6 receptor in that the former lacks the transmembrane region or both of the transmembrane region and the intracellular region. The IL-6 receptor to be used as an immunizing antigen for generating antibody may be any of the membrane-bound or the soluble IL-6 receptor, as long as the objective is attained.

After the gene sequence of IL-6 receptor was inserted into a known expression vector system to transform an appropriate host cell, the desired IL-6 receptor protein may be purified from the host cell or a culture supernatant thereof using a known method. The IL-6 receptor protein thus purified may be used as the immunizing antigen. Alternatively, cells that have expressed IL-6 receptor or a fusion protein of the IL-6 receptor protein and another protein may be used as the sensitizing antigen. A variant of IL-6 receptor may be used as the immunizing antigen for generating antibody, as long as the objective is attained.

E. coli that has a plasmid pIBIBSF2R containing cDNA encoding human IL-6 receptor has been internationally deposited under the provisions of the Budapest Treaty as HB101-pIBIBSF2R on January 9, 1989 with the National Institute of Bioscience and Human Technology, Agency of Industrial Science and Technology, of 1-3, Higashi 1-chome, Tsukuba-shi, Ibaraki, Japan, as the accession number FERM BP-2232.

### 3. Preparation of antibody-producing hybridoma

Hybridomas that produce monoclonal antibodies may be prepared using an essentially known method as follows: Thus, IL-6 receptor is used as an immunizing antigen in a conventional immunizing method for immunization. The immune cells thus obtained are fused with known parent cells and then monoclonal antibody-producing cells are screened using screening method to generate hybridomas.

Though the mammals to be immunized with an immunizing antigen are not specifically limited, they are preferably selected in consideration of their compatibility with the parent cell for use in cell fusion. They generally include rodents, lagomorphs, and primates. Rodents include mice, rats, hamsters, and the like. Lagomorphs include, for example, rabbits.
Primates include, for example, monkeys. As monkeys, catarrhines (Old-World monkeys) such as cynomolgi (crabeating macaque), rhesus monkeys, sacred baboons, chimpanzees etc. are used.

Immunization of animals with an immunizing antigen is carried out using a known method. A general method, for example, involves the intraperitoneal or subcutaneous administration of an immunizing antigen to a mammal. Specifically, an immunizing antigen which has been diluted and suspended in an appropriate amount of phosphate buffered saline (PBS) or physiological saline etc. is mixed, as desired, with an appropriate amount of a conventional adjuvant, for example Freund's complete adjuvant. After being emulsified, it is preferably administered to a mammal for several times every 4 to 21 days. Alternatively a suitable carrier may be used at the time of immunization by the immunizing antigen.

After immunization and the confirmation of the increase in the desired antibody levels in the serum, the immune cells such as lymphatic cells and spleen cells are taken out from the mammal and are subjected to cell fusion, in which preferred immune cells that are subjected to cell fusion include in particular the spleen cells.

The mammalian myeloma cells as the other parent cells which are subjected to cell fusion with the above-mentioned immune cells preferably include various known cell lines such as P3X63Ag8.653) (J. Immunol. (1979) 123: 1548-1550), P3X63Ag8U.1 (Current Topics in Microbiology and Immunology (1978) 81: 1-7), NS-1 (Kohler, G. and Milstein, C., Eur. J. Immunol. (1976) 6: 511-519), MPC-11 (Margulies, D.H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I. S., J. Exp. Med. (1978) 148: 313-323), R210 (Galfre, G. et al., Nature (1979) 277: 131-133) and the like.

Cell fusion between the above immune cells and the myeloma cells may be essentially conducted in accordance with a known method such as is described in Milstein et al. (Kohler, G. and Milstein, C., Methods Enzymol. (1981) 73: 3-46) and the like.

More specifically, the above cell fusion is carried out in the conventional nutrient broth in the presence of, for example, a cell fusion accelerator. As the cell fusion accelerator, for example, polyethylene glycol (PEG), Sendai virus (HVJ) and the like may be used, and, in addition, an adjuvant such as dimethyl sulfoxide etc. may be added as desired to enhance efficiency of the fusion.

The preferred ratio of the immune cells and the myeloma cells to be used is, for example, 1 to 10 times more immune cells than the myeloma cells. Examples of culture media to be used for the above cell fusion include RPMI1640 medium and MEM culture medium suitable for the growth of the above myeloma cell lines, and the conventional culture medium used for this type of cell culture and, besides, a serum supplement such as fetal calf serum (FCS) may be added.

In cell fusion, predetermined amounts of the above immune cells and the myeloma cells are mixed well in the above culture liquid, to which a PEG solution previously heated to about 37°C, for example a PEG solution with a mean molecular weight of about 1000 to 6000, is added at a concentration of 30 to 60% (w/v) and mixed to obtain the desired fused cells (hybridomas). Then by repeating the sequential addition of a suitable culture medium and centrifugation to remove the supernatant, cell fusion agents etc. which are undesirable for the growth of the hybridoma can be removed.

Said hybridoma is selected by culturing in a conventional selection medium, for example, the HAT culture medium (a culture liquid containing hypoxanthine, aminopterin, and thymidine). Culturing in said HAT culture medium is continued generally for a period of time sufficient to effect killing of the cells other than the desired hybridoma (non-fusion cells), generally several days to several weeks. Then, the conventional limiting dilution method is conducted in which the hybridomas that produce the desired antibody are screened and cloned.

In addition to obtaining the above hybridoma by immunizing an animal other than the human with an antigen, it is also possible to sensitize human lymphocytes in vitro with the desired antigen protein or desired antigen-expressing cells, and the resulting sensitized B lymphocytes are fused with a human myeloma cell for example U266, to obtain the desired human antibody having the activity of binding to the desired antigen or desired antigen-expressing cells (see Japanese Post-examined Patent Publication (Kokoku) No. 1-59878). Furthermore, a transgenic animal having a repertoire of all human antibody genes can be immunized with an antigen or antigen-expressing cells to obtain the desired human antibody in the method described above (International Patent Publications WO 93-12227, WO 92-03918, WO 94-02602, WO 94-25585, WO 96-34096 and WO 96-33735).

The monoclonal antibody-producing hybridomas thus constructed can be subcultured in a conventional culture medium, or can be stored for a prolonged period of time in liquid nitrogen.

In order to obtain a monoclonal antibody from said hybridoma, there can be mentioned a method in which said hybridoma is cultured in a conventional method and an antibody is obtained as a supernatant, or a method in which the hybridoma is administered to and grown in a mammal compatible with said hybridoma and the antibody is obtained as the ascites. The former method is suitable for obtaining high-purity antibodies, whereas the latter is suitable for a large scale production of antibodies.

Specifically the hybridoma producing an anti-IL-6 receptor antibody can be constructed using the method disclosed in Japanese Unexamined Patent Publication (Kokai) 3-139293. It can be conducted by a method in which the PM-1 antibody-producing hybridoma that was internationally deposited under the provisions of the Budapest Treaty as FERM BP-2998 on July 10, 1990 with the National Institute of Bioscience and Human Technology, Agency of Industrial Science and Technology, of 1-3, Higashi 1-chome, Tsukuba-shi, Ibaraki, Japan, is intraperitoneally injected to BALB/c mice (produced by CLEA Japan) to obtain the ascites from which the PM-1 antibody is purified, or a method in which said hybridoma is cultured in a.suitable culture medium such as the RPMI1640 medium containing 10% bovine fetal serum and 5% MB-Condimed H1 (manufactured by Boehringer Mannheim), the hybridoma SFM medium (manufactured by GIBCO-BRL), the PFHM-II medium (manufactured by GIBCO-BRL) and the like, and the PM-1 antibody can be purified from the supernatant.

In addition to generating a hybridoma to produce antibody, immune cells that produce the desired antibody, for example the immunized lymphocytes that were immortalized with an oncogene, may be used to obtain the antibody.

### 4. Recombinant antibody

A recombinant antibody, which is produced by the recombinant gene technology in which an antibody gene is cloned from a hybridoma and integrated into a suitable vector which was then introduced into a host, can be used in the present invention as monoclonal antibody (see, for example, Carl, A.K., Borrebaeck, and James, W. Larrick, THERAPEUTIC MONOCLONAL ANTIBODIES, published in the United Kingdom by MACMILLAN PUBLISHERS LTD. 1990).

Specifically, mRNA encoding a variable region (V) of a desired antibody is isolated from a hybridoma producing a desired antibody or antibody-producing immune cells, for example the immunized lymphocytes that were immortalized with an oncogene. The isolation of mRNA is conducted by preparing total RNA using, for example, a known method such as the guanidine ultracentrifuge method (Chirgwin, J.M. et al., Biochemistry (1979) 18, 5294-5299), the AGPC method (Chomczynski, P. et al., Analytical Biochemistry (1987) 162, 156-159), and then mRNA is purified from the total RNA using an mRNA. Purification kit (manufactured by Pharmacia) and the like. Alternatively, mRNA can be directly prepared using the Quick Prep mRNA Purification Kit (manufactured by Pharmacia).

cDNA for the V region of antibody may be synthesized from the mRNA thus obtained using a reverse transcriptase. cDNA may be synthesized using an AMV Reverse Transcriptase First-strand cDNA Synthesis Kit and the like. Alternatively, for the synthesis and amplification of cDNA, a 5'-Ampli FINDER RACE Kit (manufactured by Clontech) and the 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) that employs polymerase chain reaction (PCR) may be used. The desired DNA fragment is purified from the PCR product obtained and may be ligated to a vector DNA. Moreover, a recombinant vector is constructed therefrom and then is introduced into E. coli etc., from which colonies are selected to prepare a desired recombinant vector. The nucleotide sequence of the desired DNA may be confirmed by a known method such as the dideoxy method.

Once the DNA encoding the V region of the desired antibody has been obtained, it may be ligated to DNA encoding a constant region (C region) of the desired antibody, which is then integrated into an expression vector. Alternatively, the DNA encoding the v region of the antibody may be integrated into an expression vector which already contains DNA encoding the C region of the antibody.

In order to produce an antibody for use in the present invention, an antibody gene is integrated, as described below, into an expression vector so as to be expressed under the control of the expression regulatory region, for example an enhancer and/or a promoter. Subsequently, the expression vector may be transformed into a host cell and the antibody can then be expressed therein.

The expression of the antibody gene may be conducted by separately integrating the heavy chain (H chain) or the light chain (L chain) into a different expression vector followed by the simultaneous transformation of the host, or by integrating the DNA encoding the H chain and the L chain into a single expression vector followed by the transformation of the host (see International Patent Publication WO 94-11523).

### 5. Altered antibody

In accordance with the present invention, artificially altered recombinant antibody such as chimeric antibody and humanized antibody can be used for the purpose of lowering heterologous antigenicity against humans. These altered antibodies can be produced using known methods.

Chimeric antibody can be obtained by ligating a thus obtained DNA encoding a V region of antibody to a DNA encoding a C region of human antibody, which is then integrated into an expression vector and introduced into a host for production of an antibody therein (see European Patent Publication EP 125023, and International Patent Publication WO 92-19759). Using this known method, chimeric antibody useful for the present invention can be obtained.

For example, a plasmid that contains a DNA encoding an L chain V region or an H chain V region of a chimeric PM-1 antibody was designated as pPM-k3 or pPM-hl, respectively, and E. coli having the plasmid has been internationally deposited under the provisions of the Budapest Treaty as NCIMB 40366 and NCIMB 40362, respectively, on February 11, 1991 with the National Collections of Industrial and Marine Bacteria Limited (see International Japanese Patent Publication WO 92-19759).

Humanized antibody, which is also called reshaped human antibody, has been made by transplanting the complementarity determining regions (CDRs) of an antibody of a mammal other than the human, for example mouse antibody, into CDRs of a human antibody. The general recombinant DNA technology for preparation of such antibodies is also known (see European Patent Publication EP 125023 and International Patent Publication WO 92-19759).

Specifically, a DNA sequence wherein CDRs of a mouse antibody are ligated with framework regions (FRs) of a human antibody is synthesized from several divided oligonucleotides having sections overlapping with one another at the ends thereof. The DNA thus obtained is ligated to a DNA encoding a C region of a human antibody and then is integrated into an expression vector, which is then introduced into a host for antibody production (see European Patent Publication EP 239400 and International Patent Publication WO 92-19759).

FRs of a human antibody joined through CDRs are selected so that the CDRs form a favorable antigen binding site. When desired, amino acids in the FRs of the antibody V region may be substituted so that the CDR of reshaped human antibody may form an appropriate antigen biding site (Sato, K. et al., Cancer Res. (1993) 53, 851-856).

For chimeric antibody or humanized antibody, a C region of human antibody is used. As the preferred C region of human antibody, there can be mentioned Cγ; and, for example, Cγ1, Cγ2, Cγ3, and Cγ4 can be used. The C region of human antibody may be modified to improve the stability of antibody or the production thereof.

Chimeric antibody consists of V regions of an antibody derived from a mammal other than the human and C regions derived from a human antibody, whereas a humanized antibody consists of CDRs of an antibody derived from a mammal other than the human and FRs and C regions derived from a human antibody. Accordingly, antigenicity thereof in the human body has been reduced so that they are useful as an active ingredient of therapeutic agents of the present invention.

A preferred embodiment of the humanized antibody for use in the present invention includes humanized PM-1 antibody (see International Patent Publication WO 92-19759).

### 6. Antibody fragments and modified antibody

Antibodies for use in the present invention may be antibody fragments or modified antibodies as long as they are preferably used in the present invention. For example, as fragments of antibody, there may be mentioned Fab, F(ab')₂, Fv or single-chain Fv (scFv) in which Fv's of H chain and L chain are ligated via a suitable linker.

Specifically antibody is treated with an enzyme, for example, papain or pepsin, to produce antibody fragments, or gene encoding an antibody fragment is constructed, and then introduced into an expression vector, which is then expressed in a suitable host cell (see, for example, Co, M. S. et al., J. Immunol. (1994) 152, 2968-2976; Better, M. and Horwitz, A.H., Methods in Enzymology (1989) 178, 476-496, Academic Press, Inc.; Pluecktrun, A. and Skerra, A., Methods in Enzymology (1989) 178, 476-496, Academic Press, Inc.; Lamoyi, E., Methods in Enzymology (1989) 121, 652-663; Rousseaux, J. et al., Methods in Enzymology (1989) 121, 663-669; Bird, R.E. et al., TIBTECH (1991) 9, 132-137).

scFv can be obtained by ligating a V region of an H chain and a V region of an L chain of an antibody (see, International Patent Publication WO 88-09344). In a scFv, a V region of an H chain and a V region of an L chain are preferably ligated via a linker, preferably a peptide linker (Huston, J.S. et al., Proc. Natl. Acad. Sci. U.S.A. (1988) 85, 5879-5883). The V region of H chain and the V region of L chain in the scFv may be derived from any of the above-mentioned antibodies. As a peptide linker for linking V regions, any single-chain peptide comprising, for example, for example one comprising 12 to 19 amino acid residues may be used (see, United States Patent No. US 5525491).

DNA encoding scFv can be obtained using DNA encoding an H chain or an H chain V region of the above antibody and DNA encoding an L chain or an L chain V region of the above antibody as the template by amplifying the portion of the DNA encoding the desired amino acid sequence among the above sequences by the PCR technique with the primer pair specifying the both ends thereof, and by further amplifying the combination of DNA encoding the peptide linker portion and the primer pair which defines that both ends of said DNA be ligated to the H chain and the L chain, respectively.

Once DNA encoding scFv is constructed, an expression vector containing it and a host transformed with said expression vector can be obtained by the conventional methods, and scFv can be obtained using the resultant host by the conventional methods.

An antibody fragment can be produced by obtaining a gene therefor in a similar manner to that mentioned above and by allowing it to be expressed in a host. "Antibody" as used in the claim of the present application encompasses these antibody fragments.

As modified antibodies, antibodies associated with various molecules such as polyethylene glycol (PEG) can be used. "Antibody" as used in the claim of the present application encompasses these modified antibodies. These modified antibodies can be obtained by chemically modifying the antibodies thus obtained. These methods have already been established in the art.

### 7. Expression and production

Antibody genes constructed as described above may be expressed and antibodies are obtained in a known method. In the case of mammalian cells, expression may be accomplished using a vector containing a conventionally used useful promoter/enhancer, an antibody gene to be expressed, and DNA in which the poly A signal has been operably linked at 3' downstream thereof or a vector containing said DNA. Examples of the promoter/enhancer include the human cytomegalovirus immediate early promoter/enhancer.

Additionally, as the promoter/enhancer which can be used for expression of antibody for use in the present invention, there are viral promoters/enhancers such as retrovirus, polyoma virus, adenovirus, and simian virus 40 (SV40), and promoters/enhancers derived from mammalian cells such as human elongation factor 1α (HEF1α).

For example, expression may be readily accomplished by the method of Mulligan et al. (Nature (1979) 277, 108) when SV40 promoter/enhancer is used, or by the method of Mizushima et al. (Nucleic Acids Res. (1990) 18, 5322) when HEF1α promoter/enhancer is used.

In the case of E. coli, expression may be conducted by operably linking a commonly used useful promoter, a signal sequence for antibody secretion, and an antibody gene to be expressed, followed by expression thereof. As the promoter, for example, there can be mentioned lacz promoter and araB promoter. The method of Ward et al. (Nature (1098) 341, 544-546; FASEB J. (1992) 6, 2422-2427) may be used when lacz promoter is used, and the method of Better et al. (Science (1988) 240, 1041-1043) may be used when araB promoter is used.

As the signal sequence for antibody secretion, when produced in the periplasm of E. coli, the pelB signal sequence (Lei, S. P. et al., J. Bacteriol. (1987) 169, 4379) can be used. After separating an antibody produced in the periplasm, the structure of the antibody is appropriately refolded before use (see, for example, International Patent Publication WO 96/30394, and Japanese Post-examined Patent Publication (Kokoku) No. 7-93879).

As an origin of replication, there can be used those derived from SV40, polyoma virus, adenovirus, bovine papilloma virus (BPV) and the like. Furthermore, for the amplification of the gene copy number in a host cell system, expression vectors can include as a selectable marker an aminoglycoside transferase (APH) gene, a thymidine kinase (TK) gene, an E. coli xanthine guaninephosphoribosyl transferase (Ecogpt) gene, a dihydrofolate reductase (dhfr) gene or the like.

For the production of antibody for use in the present invention, any production system can be used. The production system of antibody preparation comprises an in vitro or an in vivo production system. As the in vitro production system, there can be mentioned a production system which employs eukaryotic cells and the production system which employs prokaryotic cells.

When the eukaryotic cells are used, there are the production systems which employ animal cells, plant cells, and fungal cells. Known animal cells include (1) mammalian cells such as CHO cells (J. Exp. Med. (1995) 108, 945), COS cells, myeloma cells, baby hamster kidney (BHK) cells, HeLa cells, and Vero cells, (2) amphibian cells such as Xenopus oocytes (Valle, et al., Nature (1981) 291, 358-340), or (3) insect cells such as sf9, sf21, and Tn5. As the CHO cells, dhfr-CHO (Proc. Natl. Acad. Sci. U.S.A. (1968) 77, 4216-4220), a CHO cell that is deficient in the DHFR gene, and CHO K-1 (Proc. Natl. Acad. Sci. U.S.A. (1968) 60, 1275) can be preferably used.

Known plant cells include, for example, those derived from Nicotiana tabacum, which is subjected to callus culture. Known fungal cells include yeasts such as the genus Saccharomyces, more specifically the species Saccharomyces cereviceae, or filamentous fungi such as the genus Aspergillus, more specifically the species Aspergillus niger.

When the prokaryotic cells are used, there are production systems which employ bacterial cells. Known bacterial cells include Escherichia coli (E. coli), and Bacillus subtilis.

By introducing via transformation a gene for a desired antibody into these cells and culturing the transformed cells in vitro, the antibody can be obtained. Culturing is conducted in the known methods. For example, as the culture media, DMEM, MEM, RPMI1640, and IMDM can be used, and serum supplements such as fetal calf serum (FCS) may be used in combination. In addition, antibodies may be produced in vivo by implanting cells into which an antibody gene has been introduced into the abdominal cavity of an animal and the like.

As in vivo production systems, there can be mentioned those which employ animals and those which employ plants. Antibody genes are introduced into these animals or plants, and the antibodies are produced in such animals or plants, and recovered.

When animals are used, there are the production systems which employ mammals and insects.

As mammals, goats, pigs, sheep, mice, and cattle can be used (Vicki Glaser, SPECTRUM Biotechnology Applications, 1993). When mammals are used, transgenic animals can be used.

For example, an antibody gene is inserted into the middle of a gene encoding protein which is inherently produced in the milk such as goat β casein to prepare fusion genes. A DNA fragment containing a fusion gene into which an antibody gene has been inserted is injected into a goat embryo, and the embryo is introduced into a female goat. The desired antibody is obtained from the milk produced by the transgenic goat born from the goat who received the embryo, or offsprings thereof. In order to increase the amount of milk containing the desired antibody produced by the transgenic goat, hormones may be given to the transgenic goat as appropriate. (Ebert, K.M. et al., Bio/Technology (1994) 12, 699-702).

When silkworms are used as an insect, baculovirus into which a desired antibody gene has been inserted is infected to silkworms, and the desired antibody can be obtained from the body fluid of the silkworm (Susumu, M. et al., Nature (1985) 315, 592-594).

Moreover, plants, for example, tabacco, can be used. When tabacco is used, a desired antibody gene is inserted into an expression vector for plants, for example pMON 530, and then the vector is introduced into a bacterium such as Agrobacterium tumefaciens. The bacterium is then infected to tabacco such as Nicotiana tabacum to obtain the desired antibody from the leaves of the tabacco (Julian, K.-C. Ma et al., Eur. J. Immunol. (1994) 24, 131-138).

When antibody is produced in vitro or in vivo production systems, as described above, a DNA encoding an H chain or an L chain of an antibody may be separately integrated into an expression vector and the hosts are transformed simultaneously, or a DNA encoding an H chain and an L chain may be integrated into a single expression vector and the host is transformed therewith (see International Patent Publication WO 94-11523).

As methods of introducing an expression vector into a host, there can be used a known method such as the calcium phosphate method (Virolgoy (1973) 52, 456-467) and the electropolation method (EMBO J. (1982) 1, 841-845), and the like.

### 8. Separation and purification of antibody

Antibodies produced and expressed as described above can be separated from the inside or outside of the host cell and then may be purified to homogeneity. Separation and purification of the antibody for use in the present invention may be accomplished by the separation and the purification methods conventionally used for protein purification. These methods include, but are not limited to, chromatography columns such as affinity chromatography, filtration, ultrafiltration, salting-out, dialysis and the like, from which methods can be selected and combined as appropriate for separation and purification (Antibodies: A Laboratory Manual, Ed Harlow and David Lane, Cold Spring Harbor Laboratory, 1988).

As columns for use in affinity chromatography, there can be mentioned Protein A column and Protein G column. Examples of the carriers used in the Protein A column are Hyper D, POROS, Sepharose F. F. (Pharmacia) and the like.

As chromatography other than the above-mentioned affinity chromatography, there can be mentioned, for example, ion exchange chromatography, hydrophobic chromatography, gel-filtration, reverse phase chromatography, adsorption chromatography, and the like (Strategies for Protein Purification and Characterization: A Laboratory Course Manual. Ed Daniel R. Marshak et al., Cold Spring Harbor Laboratory Press, 1986).

These chromatographies can be carried out using a liquid chromatography such as HPLC, FPLC.

### 9. Determination of antibody concentration

The concentration of antibody obtained can be determined by the measurement of absorbance or by the enzyme-linked immunosorbent assay (ELISA) and the like. Thus, when absorbance measurement is employed, the antibody for use in the present invention or a sample containing the antibody is appropriately diluted with PBS and then the absorbance is measured at 280 nm. In the case of human antibody, calculation is conducted using 1.35 OD at 1 mg/ml.

When the ELISA method is used, measurement is conducted as follows. Thus, 100 µl of goat anti-human IgG (manufactured by TAGO) diluted to 1 mg/ml in 0.1 M bicarbonate buffer, pH 9.6, is added to a 96-well plate (manufactured by Nunc), and is incubated overnight at 4°C to immobilize the antibody. After blocking, 100 µl each of appropriately diluted antibody of the present invention or a sample containing the antibody, or 100 µl of human IgG (manufactured by CAPPEL) as the concentration standard is added, and incubated at room temperature for 1 hour.

After washing, 100 µl of 5000-fold diluted alkaline phosphatase-labeled anti-human IgG (manufactured by BIO SOURCE) is added, and incubated at room temperature for 1 hour. After washing, the substrate solution is added and incubated, followed by the measurement of absorbance at 405 nm using the MICROPLATE READER Model 3550 (manufactured by Bio-Rad) to calculate the concentration of the desired antibody.

Alternatively, BIAcore (manufactured by Pharmacia) can be used for the measurement of antibody concentration.

### 10. Confirmation of the activity of antibody

The antigen binding activity, binding-inhibition activity, and neutralization activity of an IL-6 receptor antibody for use in the present invention can be evaluated using a conventionally known method. For example, IL-6 is added to a plate in which IL-6-reactive cells such as MH60.BSF2 were cultured. Then the activity can be evaluated using incorporation of ³H-thymidine into IL-6-reactive cells in the coexistence of an IL-6 antagonist as an index.

Alternatively, anti-IL-6 receptor antibody and ¹²⁵I-labeled IL-6 are added to a plate in which IL-6 receptor-expressing cells such as U266 were cultured. Then, evaluation can be effected by determining the ¹²⁵I-labeled IL-6 bound to the IL-6 receptor-expressing cells (Antibodies: A Laboratory Manual, Ed Harlow and David Lane, Cold Spring Harbor Laboratory, 1988).

Furthermore, as methods of determining the antigen binding activity of the IL-6 receptor antibody for use in the present invention, there can be used ELISA, EIA (enzymeimmunoassay), RIA (radioimmunoassay), or the fluorescent antibody method.

When ELISA is employed, for example, IL-6 receptor is added to a 96-well plate onto which antibody against IL-6 receptor, for example antibody against the C region thereof, has been immobilized, and then samples containing the desired anti-IL-6 receptor antibody, for example a culture supernatant of anti-IL-6 receptor antibody-producing cells or purified antibody, are added thereto.

Secondary antibody that recognizes the desired anti-anti-IL-6 receptor antibody labeled with an enzyme such as alkaline phosphatase is added, and the plate is incubated, and washed. Then, after adding an enzyme substrate such as p-nitrophenyl phosphate thereto and determining absorbance, the antigen-binding activity can be evaluated. A soluble IL-6 receptor may be used as the IL-6 receptor.

As methods for measuring the inhibitory activity of ligand receptor binding of the anti-IL-6 receptor antibody for use in the present invention, the conventional Cell ELISA or the ligand receptor binding assay can be used.

In the case of Cell ELISA, for example, cells expressing IL-6 receptor are cultured in a 96-well plate and then adhered, and immobilized with paraformaldehyde etc. Alternatively, membrane fractions of cells expressing IL-6 receptor are prepared and a 96-well plate on which the fractions have been immobilized is prepared. To the plate are added a sample containing the desired anti-IL-6 receptor antibody, for example a culture supernatant of anti-IL-6 receptor antibody-producing cells, purified antibody, and a radioisotope such as ¹²⁵I-labeled IL-6.

Then the plate is incubated, washed, and radioactivity is measured to determine the amount of IL-6 bound to the IL-6 receptor and thereby to evaluate the inhibition activity of ligand receptor binding of anti-IL-6 receptor antibody.

In the inhibition assay of IL-6 binding to IL-6 receptor on the cells, cells expressing IL-6 receptor are separated by means of centrifugation etc. to prepare a cell suspension. A solution of IL-6 labeled with a radioisotope such as ¹²⁵I, or a mixture of unlabeled IL-6 and labeled IL-6, and a solution containing anti-IL-6 receptor antibody whose concentration has been adjusted are added to the cell suspension. After incubating for a certain period of time, the cells are separated, and the radioactivity of the labeled IL-6 bound to the cell is measured.

For evaluation of the activity of the above antibody, BIAcore (manufactured by Pharmacia) can be used.

### 11. Confirmation of therapeutic effects

The subject disease to be treated by the preventive and/or therapeutic agents of the present invention is systemic lupus erythematosus. In order to confirm the effects accomplished by the present invention, anti-IL-6 receptor antibody may be given to a model animal that develops the pathology of human systemic lupus erythematosus.

As the animal to be used, the model animals that best manifest the pathology of human systemic lupus erythematosus are used. The model animals that best manifest the pathology of human systemic lupus erythematosus are NZB/WF1 mice. NZB/WF1 mice are a known and available model animal.

Systemic lupus erythematosus is a systemic autoimmune disease that is caused by the deposition of anti-nuclear antibody or anti-DNA antibody or their immune complexes in various organs and/or tissues, which induces inflammatory reactions thereby causing systemic autoimmune disease. The deposition in the renal glomerulus of anti-nuclear antibody or anti-DNA antibody or their immune complexes causes autoimmune lupus nephritis. Few patients with systemic lupus erythematosus exhibit an increase in IL-6 in the blood, thereby negating the involvement of IL-6 in the pathology (Peterson, E. et al., Lupus. (1996) 5, 571-576).

It has been reported that IL-6 directly acts on the mesangial cells present in the glomerulus of the kidney and thereby causes the growth of the mesangial cells, which in turn induces glomerular nephritis (Suematsu, S. et al., Proc. Natl. Acad. Sci. U.S.A. (1989) 86, 7547-7551). Lupus nephritis is caused by the mechanism in which IgG class anti-nuclear antibody or anti-DNA antibody deposits in the glomerulus and thereby causes inflammatory reactions in the glomerulus, which in turn damages the glomerulus leading to the onset of nephritis, and therefore from the viewpoint of the mechanism of onset it is distinguished from mesangial proliferative glomerulonephritis.

As shown in the Examples that follow, the suppression of IgG class anti-DNA antibody or the suppression of the excretion of urinary protein were observed by administering anti-IL-6 receptor antibody to NZB/WF1 mice. Since NZB/WF1 mice are a model animal that best reflects the pathology of human systemic lupus erythematosus, it was revealed that anti-IL-6 receptor antibody has a therapeutic effect on systemic lupus erythematosus.

Thus, the preventive and/or therapeutic agents of the present invention are useful as preventive and/or therapeutic agents for systemic lupus erythematosus. The preventive and/or therapeutic agents of the present invention are useful as preventive and/or therapeutic agents for reducing anti-DNA antibody in systemic lupus erythematosus, and as preventive and/or therapeutic agents for reducing the excretion of urinary protein in systemic lupus erythematosus.

### 12. Route of administration and pharmaceutical preparation

The preventive and/or therapeutic agents of the present invention may be administered orally or pareterally, either systemically or locally. The parenteral route may be selected from intravenous injection such as drip infusion, intramuscular injection, intraperitoneal injection, and subcutaneous injection, and the method of administration may be chosen, as appropriate, depending on the age and the conditions of the patient.

The preventive and/or therapeutic agents of the present invention may be administered at a dosage that is sufficient to treat or to block at least partially the pathological condition. For example, the effective dosage is chosen from the range of 0.01 mg to 100 mg per kg of body weight per administration. Alternatively, the dosage in the range of 1 to 1000 mg, preferably 5 to 50 mg per patient may be chosen. However, the preventive and/or therapeutic agents of the present invention are not limited to these dosages.

The timing of administration may be after the development of systemic lupus erythematosus or the agents may be administered when the onset of systemic lupus erythematosus is expected in a preventive manner to alleviate the pathological conditions after the onset.

The period of administration may be selected as appropriate depending on the age and condition of the patient.

The preventive or therapeutic agents of the present invention may contain pharmaceutically acceptable carriers or additives depending on the route of administration. Examples of such carriers or additives include water, a pharmaceutical acceptable organic solvent, collagen, polyvinyl alcohol, polyvinylpyrrolidone, a carboxyvinyl polymer, carboxymethyl cellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, propylene glycol, polyethylene glycol, Vaseline, paraffin, stearyl alcohol, searic acid, human serum albumin (HSA), mannitol, sorbitol, lactose, a pharmaceutically acceptable surfactant and the like. Additives used are chosen from, but not limited to, the above or combinations thereof depending on the dosage form.

### Examples

The present invention will now be explained in more details with reference to the working examples, reference examples, and experiments. It should be noted, however, that the present invention is not limited to them in any way.

### Reference example 1. Preparation of human soluble IL-6 receptor

Soluble IL-6 receptor was prepared by the PCR method using a plasmid pBSF2R.236 containing cDNA that encodes IL-6 receptor obtained according to the method of Yamasaki et al., Science (1988) 241, 825-828. Plasmid pBSF2R.236 was digested with a restriction enzyme Sph I to obtain a cDNA of IL-6 receptor, which was then inserted into mp18 (manufactured by Amersham). Using a synthetic oligoprimer designed to introduce a stop codon into the cDNA of IL-6 receptor, a mutation was introduced into the cDNA of IL-6 receptor by the PCR method using an in vitro Mutagenesis System (manufactured by Amersham). The procedure resulted in the introduction of a stop codon to the amino acid at position 345, and gave a cDNA encoding soluble IL-6 receptor.

In order to express the cDNA of soluble IL-6 receptor in CHO cells, it was ligated to plasmid pSV (manufactured by Pharmacia) to obtain plasmid pSVL344. The cDNA of soluble IL-6 receptor that was cleaved with Hind III-Sal I was inserted to plasmid pECEdhfr containing the cDNA of dhfr to obtain plasmid pECEdhfr344 that can be expressed in the CHO cells.

Ten µg of plasmid pECEdhfr344 was transfected to a dhfr-CHO cell line DXB-11 (Urland et al., Proc. Natl. Acad. Sci. U.S.A. (1980) 77, 4216-4220) by the calcium phosphate method (Chen et al., Mol. Cell. Biol. (1987) 7, 2745-2751). The transfected CHO cells were cultured in a nucleoside-free α MEM selection medium containing 1 mM glutamin, 10% dialyzed FCS, 100 U/ml penicillin, and 100 µg/ml streptomycin.

The selected CHO cells were screened by the limiting dilution method to obtain a single CHO cell clone. The CHO cell clone was amplified in 20 nM - 200 nM methotrexate (MTX) to obtain a CHO cell line 5E27 that produces human soluble IL-6 receptor. The CHO cell line 5E27 was cultured in an Iscov-modified Dulbecco's medium (IMDM, manufactured by Gibco) containing 5% FBS. The culture supernatant was collected and the concentration of soluble IL-6 receptor in the culture supernatant was determined by ELISA.

### Reference example 2. Preparation of human anti-IL-6 receptor antibody

Anti-IL-6 antibody MT18 prepared by the method of Hirata et al. (J. Immunol., 143, 2900-2906, 1989) was bound to CNBr-activated Sepharose 4B (manufactured by Pharmacia Fine Chemicals, Piscataway, NJ) according to the attached regimen and IL-6 receptor (Science (1988) 241, 825-8258) was purified. A human myeloma cell line U266 was solubilized with 1 mM p-para-aminophenyl methane sulfonyl fluoride hydrochloride (manufactured by Wako Chemicals) containing 1% digitonin (manufactured by Wako Chemicals), 10 mM triethanolamine (pH 7.8) and 0.15 M NaCl (manufactured by Wako Chemicals) (digitonin buffer), and mixed with MT18 antibody bound to Sepharose 4B beads. Then, the beads were washed six times with the digitonin buffer, to prepare the partially purified IL-6 receptor.

BALB/c mice were immunized four times every ten days with the above partially purified IL-6 receptor obtained from 3 x 10⁹ U266 cells, and then a hybridoma was prepared using a standard method. The hybridoma culture supernatant from the growth-positive well was tested for its activity of binding to IL-6 receptor according to the method described below. 5 x 10⁷ U266 cells were labeled with ³⁵S-methionine (2.5 mCi) and were solubilized with the above digitonin buffer. The solubilized U266 cells were mixed with a 0.04 ml volume of MT18 antibody bound to Sepharose 4B beads, and then were washed six times with the digitonin buffer. ³⁵S-methionine-labeled IL-6 receptor was eluted with 0.25 ml of the digitonin buffer (pH 3.4) and was neutralized in 0.025 ml of 1M Tris (pH 7.4).

0.05 ml of the hybridoma culture supernatant was mixed with 0.01 ml of Protein G Sepharose (manufactured by Pharmacia). After washing, Sepharose was incubated with 0.005 ml ³⁵S-labeled IL-6 receptor solution prepared as described above. The immunoprecipitate was analyzed by SDS-PAGE to investigate the hybridoma culture supernatant that reacts with IL-6 receptor. As a result, the reaction-positive hybridoma clone PM-1 was established. The antibody produced from the hybridoma PM-1 has a subtype of IgGlκ.

The inhibitory activity of IL-6 binding of the antibody produced by the hybridoma PM-1 to human IL-6 receptor was studied using the human myeloma cell line U266. A human recombinant IL-6 was prepared from E. coli (Hirano et al., Immunol. Lett., 17, 41), and was labeled with ¹²⁵I using the Bolton-Hunter reagent (New England Nuclear, Boston, MA) (Taga, T. et al., J. Exp. Med. (1987) 166, 967-981). 4 x 10⁵ U266 cells were cultured with the culture supernatant of 70% (v/v) hybridoma PM-1 together with 14,000 cpm of ¹²⁵I-labeled IL-6 in the presence of a 100-fold excess of unlabeled IL-6 for one hour at room temperature. Seventy µl of the sample was layered on 300 µl FCS in a 400 µl microfuge polyethylene tube. After centrifugation, the radioactivity of the cell was determined.

The result revealed that the antibody produced by the hybridoma PM-1 inhibits the binding of IL-6 to IL-6 receptor.

### Reference example 3. Preparation of mouse anti-IL-6 receptor antibody

A monoclonal antibody directed against mouse IL-6 receptor was prepared according to the method described in Saito, et al., J. Immunol. (1993) 147, 168-173.

The CHO cells that produce mouse soluble IL-6 receptor were cultured in the IMDM culture liquid containing 10% FCS. From the culture supernatant, mouse soluble IL-6 receptor was purified using mouse soluble IL-6 receptor RS12 (see Saito, et al., supra) and an affinity column fixed to Affigel 10 gel.

The mouse soluble IL-6 receptor (50 µg) thus obtained was mixed with Freund's complete adjuvant, which was then injected to the abdomen of Wistar rats (Japan Charles River). From 2 weeks later the animals were boosted with Freund's incomplete adjuvant. On day 45, the rats were sacrificed, and about 2 x 10⁸ spleen cells were fused with 1 x 10⁷ mouse myeloma cells P3U1 using 50% PEG1500 (manufactured by Boehringer Mannheim) according to the conventional method, and then were screened by the HAT culture medium.

After the culture supernatant was added to the plate coated with rabbit anti-rat IgG antibody (manufactured by Cappel), mouse soluble IL-6 receptor was reacted. Subsequently, using rabbit anti-mouse IL-6 receptor antibody and alkaline phosphatase-labeled sheep anti-rabbit IgG, hybridomas producing antibody directed against mouse soluble IL-6 receptor were screened by ELISA. After antibody production was confirmed, the hybridoma clones were subscreened twice to obtain a single hybridoma clone. The clone was designated as MR16-1.

The neutralizing activity of the antibody produced by the hybridoma on signal transduction of mouse IL-6 was examined by ³H-thymidine incorporation using MH60.BSF2 cells (Matsuda et al., J. Immunol. (1988) 18, 951-956). To a 96-well plate, MH60.BSF2 cells were prepared at 1 x 10⁴ cells/200 µl/well. To the plate were added mouse IL-6 and MR16-1 antibody or RS12 antibody at 12.3 to 1000 ng/ml, and then were cultured at 37°C and 5% CO₂ for 44 hours and then 1 µCi/well of ³H-thymidine was added. After 4 hours, the incorporation of ³H-thymidine was measured. As a result, MR16-1 antibody suppressed the incorporation of ³H-thymidine of the MH60.BSF2 cells.

Thus, it was demonstrated that the antibody produced by the hybridoma MR16-1 inhibits the binding of IL-6 to IL-6 receptor.

### Reference example 4. Preparation of humanized PM-1 antibody

Humanized PM-1 antibody was obtained according to the method described in International Patent Publication WO 92-19759. From the hybridoma PM-1 generated in Reference example 2, total RNA was prepared which was then used to synthesize a single strand cDNA. DNA encoding the variable region (V region) of mouse PM-1 antibody was amplified by the polymerase chain reaction (PCR) method. From the PCR, primers described in Jones, S. T. et al., Bio/Technology (1991) 9, 88-89, were used.

The PCR-amplified DNA fragments were purified to obtain a DNA fragment that contains a gene encoding the V region of mouse κ type L chain and a DNA fragment that contains a gene encoding the V region of mouse γ type H chain. These DNA fragments were ligated to plasmid pUC19, which was then introduced into competent cells of E. coli DH5α to obtain an E. coli transformant. From this transformant the above plasmid was obtained, and the nucleotide sequence of the cDNA coding region in the plasmid was determined by a conventional method, and the complementarity determining regions (CDR's) of each V region were determined.

In order to construct a vector expressing chimeric PM-1 antibody, cDNA encoding the respective region of the κ type L chain and H chain of the mouse PM-1 antibody was inserted into an expression vector for HCVM. After confirming that the produced chimeric PM-1 antibody has a correct antigen binding activity, the CDR-grafting method was used to construct humanized PM-1 antibody. By substituting amino acids in the framework region (FR), a humanized PM-1 antibody having an antigen binding activity almost equal to the chimeric antibody was constructed.

In order to express the gene encoding the thus obtained L chain and H chain of the humanized PM-1 antibody in mammalian cells, each gene was introduced into a vector containing the human elongation factor 1α (HEF-1α). By simultaneously transforming these expression vectors in CHO cells, CHO cell line that produces the humanized PM-1 antibody was established. The humanized PM-1 antibody obtained was confirmed to have an antigen binding activity to human IL-6 receptor by the ELISA. Furthermore, the humanized PM-1 antibody inhibited the binding of human IL-6 to IL-6 receptor to a degree equal to that of the mouse PM-1 antibody and the chimeric PM-1 antibody.

### Example 1.

MR16-1 and KH-5 (anti-DNP antibody) are rat IgG and when continuously given it is likely to cause the production of antibody against rat IgG and shock symptoms. Immune tolerance against these antibodies was induced using the method described in Finck, B. K. et al., J. Clin. Invest. (1994) 94, 585-591, before the start of MR16-1 and KH-5 administration.

Thus, 1 mg of anti-CD4 antibody (GK1.5) was intraperitoneally given for three consecutive days to 13-week-old female NZB/W F1 mice (ten mice per group, except 9 mice for vehicle administration), and 0.5 mg of MR16-1 or KH-5 was intraperitoneally given simultaneously with the second administration of anti-CD4 antibody. Thereafter, MR16-1 (0.5 mg), an equal amount of KH-5 as the control antibody, and saline as the vehicle control were intraperitoneally given once per week till the animals become 64 weeks old, with regular measurement of the urinary protein excreted and the blood levels of anti-DNA antibody.

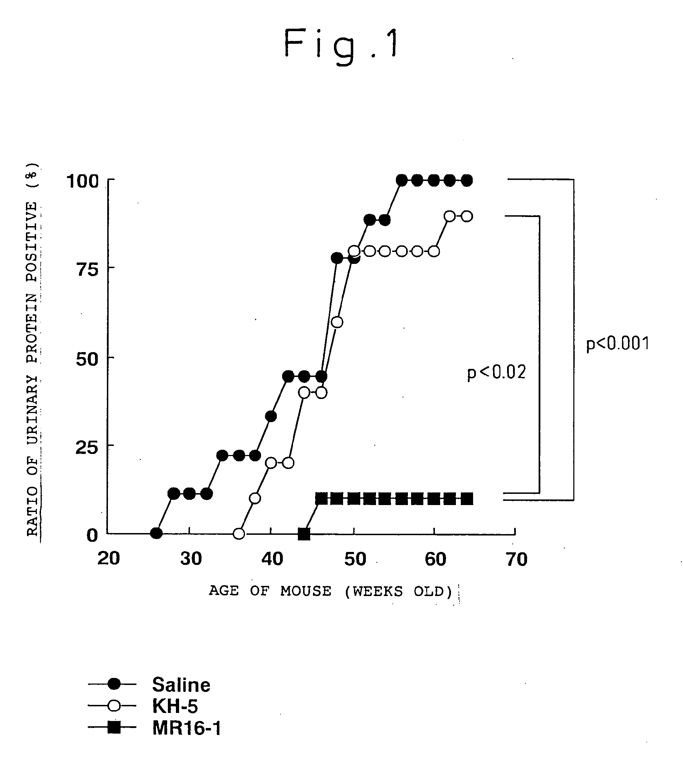

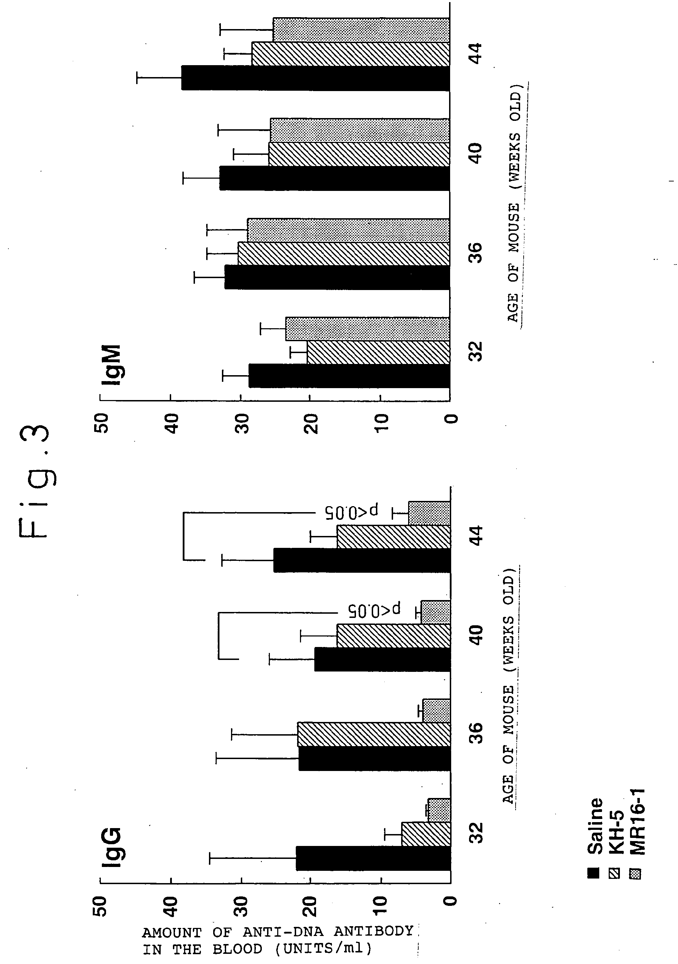

The amount of immunoglobulins in the blood was also determined. The measurement of the amount of urinary protein was conducted using Combisticks paper (manufactured by Sankyo), and individuals having more than 100 mg/dl protein were considered positive. The results are shown in Figure 1. The amount of anti-DNA antibody in the blood was determined by the ELISA method. The results (mean +/- standard error) are shown in Figure 3. The amount of immunoglobulins in the blood was determined by the radial immunodiffusion assay. The results (mean +/- standard error) are shown in Figure 1.

**Table 1 The amount of immunoglobulins in the blood at 32 weeks old (mg/ml)**

| | IgG1 | IgG2a | IgG3 | IgM | IgA |
|---|---|---|---|---|---|
| Saline | 1.24 ± 0.11 | 6.44 ± 1.38 | 2.25 ± 0.37 | 1.40 ± 0.14 | 1.13 ± 0.06 |
| KH-5 | 1.14 ± 0.09 | 4.18 ± 0.57 | 1.82 ± 0.27 | 1.20 ± 0.09 | 1.18 ± 0.11 |
| MR16-1 | 0.84 ± 0.14 | 3.21 ± 0.46 | 1.23 ± 0.15 | 1.21 ± 0.13 | 1.15 ± 0.04 |

Table 1 shows the effect of MR16-1 on the amount of immunoglobulins in the blood. In the table, the results for IgG subclass, IgM and IgA are those at 32 weeks old.

Thus, the excretion of urinary protein begins to be observed at 28 weeks old for the vehicle administration group, and at 38 weeks old for the KH-5 administration group. At 64 weeks old, the time of completion of the experiment, all mice of the vehicle administration group and 90% of the mice of the KH-5 administration group were positive, whereas in the MR16-1 administration group the timing of the appearance of urinary protein was markedly delayed and the incidence of the disease was also markedly suppressed.

With regard to the days of survival, MR16-1 clearly prolonged the days of survival and only one mouse died by 64 weeks old. In the amount of anti-DNA antibody, the production of IgG class anti-DNA antibody was markedly suppressed in the MR16-1 administration group but no effects were seen in the antibody of IgM class. In the amount of immunoglobulins in the blood, the amounts of IgG1, IgG2, and IgG3 were decreased in the MR16-1 administration group, but no effects were seen in the amounts of IgM and IgA.

The above results revealed that anti-IL-6 receptor antibody is useful as a preventive and/or therapeutic agent for systemic lupus erythematosus.

In order to inhibit the biological activity of IL-6, the use of anti-IL-6 antibody and anti-IL-6 receptor antibody is considered, but it is believed that anti-IL-6 receptor antibody has a more potent therapeutic effect than anti-IL-6 antibody in systemic lupus erythematosus.

When anti-IL-6 receptor antibody was used in the present invention, the onset of nephritis was not observed except in one mouse, whereas when anti-IL-6 antibody was used in the experiment of Finck, B. K. et al., J. Clin. Invest. (1994) 94, 585-591, the number of mice that developed nephritis was growing from 7 weeks old, and the tendency is likely to continue after 9 weeks old. From these results, it is estimated that the effect of anti-IL-6 antibody is insufficient as compared to that of anti-IL-6 receptor antibody.

In the Finck et al. 's report, changes in the amount of immunoglobulins in the blood included a 2% decrease in IgG1, a 12% decrease in IgG2a, and a 92% increase in IgG3, and it described that anti-IL-6 antibody had no effects on the amount of total immunoglobulins in the blood. On the other hand, in the experiment in which the anti-IL-6 receptor antibody of the present invention was used, there were a 27% decrease in IgG1, a 23% decrease in IgG, and a 32% decrease in IgG3. From these results also, it is estimated that anti-IL-6 receptor antibody has a more potent effect.

There are many reports that indicate that the administration of anti-IL-6 antibody to humans or mice causes a marked increase in the concentration of IL-6 in the blood (Wendling, D. et al., J. Rheumatol. (1993) 20, 259-262, Heremans, H. et al., Eur. J. Immunol. (1992) 22, 2395-2401. It has also been found that the simultaneous administration of IL-6 and anti-IL-6 antibody causes a marked increase in the activity of IL-6 (Mihara, J. et al., Immunology (1991) 74, 55-59).

In accordance with the present invention, the serum concentration of IL-6 determined for mice that received anti-IL-6 receptor antibody was below the detection limit. Thus, it suggested the possibility that the administration of anti-IL-6 receptor antibody does not increase IL-6 in the blood. From these results also, it is believed that anti-IL-6 receptor antibody is more excellent than anti-IL-6 antibody.

### Industrial Applicability

In accordance with the present invention, it was shown that anti-IL-6 receptor antibody has a therapeutic effect for systemic lupus erythematosus. Thus, anti-IL-6 receptor antibody is useful as a preventive and/or a therapeutic agent for systemic lupus erythematosus.

Anti-IL-6 receptor antibody is also useful as a preventive and/or a therapeutic agent for reducing anti-DNA antibody in systemic lupus erythematosus and for reducing the excretion of urinary protein in systemic lupus erythematosus.

Reference to the microorganisms deposited under the Patent Cooperation Treaty, Rule 13-2, and the name of the Depository Institute:

### Depository Institute

Name: the National Institute of Bioscience and Human Technology, Agency of Industrial Science and Technology

Address: 1-3, Higashi 1-chome, Tsukuba-shi, Ibaraki, Japan
Organism (1)
   Identification: Rat-mouse hybridoma MR16-1
   Deposition number: FERM BP-5875
   Deposition date: March 13, 1997
Organism (2)
   Identification: HB 101-pIBIBSF2R
   Deposition number: FERM BP-2232
   Deposition date: January 9, 1989
Organism (3)
   Identification: PM1
   Deposition number: FERM BP-2998
   Deposition date: July 12, 1989
Depository organ
   Name: National Collection of Industrial and Marine
Bacteria Limited
   Address: 23 st Machar Drive Aberdeen AB2 IRY
Organism (4)
   Identification: Escherichia coli DH5α-pPM-k3
   Deposition number: NCIMB 40366
   Deposition date: February 12, 1991
Organism (5)
   Identification: Escherichia coli DH5α-pPM-h1
   Deposition number: NCIMB 40362
   Deposition date: February 12, 1991

## Claims

1. A preventive and/or therapeutic agent for systemic lupus erythematosus comprising an interleukin-6 (IL-6) receptor antibody as an active ingredient.

2. A preventive and/or therapeutic agent for autoimmune nephritis in systemic lupus erythematosus.

3. A preventive and/or therapeutic agent according to claim 2 wherein the autoimmune nephritis is lupus nephritis.

4. The preventive and/or therapeutic agent according to any of claims 1 to 3 wherein the anti-IL-6 receptor antibody is anti-human IL-6 receptor antibody.

5. The preventive and/or therapeutic agent according to any of claims 1 to 4 wherein the anti-IL-6 receptor antibody is anti-IL-6 receptor monoclonal antibody.

6. The preventive and/or therapeutic agent according to any of claims 1 to 5 wherein the anti-IL-6 receptor antibody is a recombinant IL-6 receptor antibody.

7. The preventive and/or therapeutic agent according to any of claims 1 to 6 wherein the anti-IL-6 receptor antibody is PM-1 antibody.

8. The preventive and/or therapeutic agent according to any of claims 1 to 7 wherein the anti-IL-6 receptor antibody is an antibody having the constant region of human antibody.

9. The preventive and/or therapeutic agent according to any of claims 1 to 8 wherein the anti-IL-6 receptor antibody is a chimeric or humanized antibody directed against IL-6 receptor.

10. The preventive and/or therapeutic agent according to any of claims 1 to 9 wherein the anti-IL-6 receptor antibody is humanized PM-1 antibody.

11. The preventive and/or therapeutic agent for reducing anti-DNA antibody or anti-nuclear antibody in systemic lupus erythematosus, said agent comprising anti-IL-6 receptor antibody as an active ingredient.
